# EUROPEAN PATENT APPLICATION

(11) **EP 2 893 927 A1**
(43) Date of publication of application: **15.07.2015**
(21) Application number: 13833389.3
(22) Date of filing: 02.09.2013
(51) Int. Cl.: A61K 31/27, A61K 9/70, A61K 47/32, A61K 47/34, A61P 25/28

(54) **ADHESIVE SKIN PATCH**

(30) Priority: 03.09.2012 JP 2012193584; 16.01.2013 JP 2013005616
(71) Applicant: NIPRO Patch Co., Ltd., Saitama 344-0057 (JP)
(72) Inventor: KAWAMURA, Naohisa, Kasukabe-shi Saitama 344-0057 (JP); SAWADA, Hidenori, Kasukabe-shi Saitama 344-0057 (JP)
(74) Representative: Ström & Gulliksson AB
(86) International application number: PCT/JP2013/073590
(87) International publication number: WO 2014/034939

(57) **Abstract**

The purpose of the present invention is to provide a rivastigmine-containing adhesive skin patch which has excellent transdermal permeability of rivastigmine contained therein and has excellent adhesion performance. The present invention provides an adhesive skin patch comprising a support and an adhesive agent layer arranged on the support, wherein the adhesive agent layer contains at least rivastigmine and/or a pharmaceutically acceptable salt thereof and a specific acrylic adhesive agent. The adhesive skin patch according to the present invention may be composed of only the support and the adhesive agent layer. The adhesive agent layer may have a single-layered structure.

## Description

### TECHNICAL FIELD

The present invention relates to an adhesive skin patch.

### BACKGROUND ART

Rivastigmine is known as an agent for suppressing symptom progression of Alzheimer type dementia. For example, Patent Documents 1 and 2 describe rivastigmine-containing formulations. Further, as a rivastigmine-containing formulation, a rivastigmine-containing adhesive skin patch (Product name: Exelon (registered trademark)) is commercially available.
Patent Document 1: Japanese Patent No. 3820103
Patent Document 2: Japanese Unexamined Patent Application (Translation of PCT Application), Publication No. 2009-517468

### DISCLOSURE OF THE INVENTION

### Problems to be Solved by the Invention

However, in the case of the conventional rivastigmine-containing adhesive skin patch, it has been difficult to confer adhesiveness on a rivastigmine-containing drug layer. Therefore, an adhesive agent layer has been required to be separately provided in addition to the drug layer. Further, there has also been room for improvement in terms of the usage efficiency (the transdermal permeability of rivastigmine and the like) because an adhesive skin patch having a layered structure of 3 or more layers comprising a support, a drug layer, an adhesive agent layer and the like does not have sufficient compatibility with a drug, an adhesive agent and the like.

Moreover, in the case of the conventional rivastigmine-containing adhesive skin patch, it has been difficult to include rivastigmine, which is a liquid at ordinary temperature, at a high concentration in an adhesive agent layer. In addition to this, cold flow (a phenomenon in which an adhesive agent layer becomes soft and undergoes flow deformation in an environment such as ordinary temperature) might occur in an adhesive agent layer. When cold flow occurs, an adhesive agent layer may flow out of an adhesive skin patch. As a result of this, disadvantageously, the adhesive skin patch may be stuck in a packaging bag, and the adhesive skin patch may be difficult to be removed from the packaging bag.

The present invention is made in view of the above circumstances. An object of the present invention is to provide a rivastigmine-containing adhesive skin patch having excellent transdermal permeability of rivastigmine and excellent adhesiveness in which cold flow does not easily occur.

### Means for Solving the Problems

The present inventors completed the present invention after finding that the above problems can be solved by blending a specific acrylic adhesive agent as a component of a rivastigmine-containing adhesive skin patch. Specifically, the present invention provides the following.

An adhesive skin patch comprising a support and an adhesive agent layer arranged on the support, wherein
the adhesive agent layer comprises at least
rivastigmine and/or a pharmaceutically acceptable salt thereof, and
an acrylic adhesive agent,
wherein the acrylic adhesive agent comprises an adhesive agent (I) or an adhesive agent (II),
the adhesive agent (I) being an acrylic copolymer comprising at least diacetone acrylamide as a constituting component,
the adhesive agent (II) being an acrylic copolymer comprising at least acetoacetoxyalkyl (meth)acrylate as a constituting component.

The adhesive skin patch according to (1), wherein the acrylic adhesive agent comprises an adhesive agent (I) or an adhesive agent (II),
the adhesive agent (I) being a resin mixture comprising 100 parts by mass of an acrylic copolymer (A) and 0.1 to 30 parts by mass of an acrylic copolymer (B) or 0.05 to 2 parts by mass of a polyamine compound,
the acrylic copolymer (A) being an acrylic copolymer comprising alkyl (meth)acrylate as a main monomer component and 3 to 45 mass% of diacetone acrylamide as an essential monomer component, but not comprising a free carboxyl group,
the acrylic copolymer (B) being an acrylic copolymer comprising alkyl (meth)acrylate as a main monomer component and a primary amino group and/or a carboxy hydrazide group in a side chain, but not comprising a free carboxyl group,
the adhesive agent (II) being a copolymer of acetoacetoxyalkyl (meth)acrylate and one or more vinyl monomers copolymerizable with the acetoacetoxyalkyl (meth)acrylate, the copolymer not comprising a free carboxyl group.

The adhesive skin patch according to (1) or (2), comprising the support and the adhesive agent layer only.

The adhesive skin patch according to (1) or (2), wherein the adhesive agent layer is a single layer.

The adhesive skin patch according to any one of (1) to (4) used for treatment or prevention of Alzheimer type dementia.

### Effects of the Invention

The present invention provides a rivastigmine-containing adhesive skin patch having excellent transdermal permeability of rivastigmine and excellent adhesiveness in which cold flow does not easily occur.

### BRIEF DESCRIPTION OF THE DRAWINGS

Fig. 1 shows the cumulative transdermal permeation amounts of rivastigmine for the adhesive skin patches according to Examples of the present invention.
Fig. 2 shows skin irritation for the adhesive skin patches according to Examples of the present invention.
Fig. 3 shows results from cold flow resistance tests for the adhesive skin patch according to Example of the present invention.

### PREFERRED MODE FOR CARRYING OUT THE INVENTION

Below, embodiments of the present invention will be described in detail.

The present invention provides an adhesive skin patch having a support and an adhesive agent layer arranged on the support comprising a specific component such as rivastigmine. When the adhesive agent layer provided on the support adheres to the skin, rivastigmine in the adhesive agent layer is absorbed into the skin from the adhesive agent layer to show a desired therapeutic effect. Below, the components of the adhesive skin patch according to the present invention are each described.

### [Adhesive agent layer]

The adhesive agent layer in the present invention comprises at least rivastigmine and/or a pharmaceutically acceptable salt thereof and an acrylic adhesive agent as described below.

### (Rivastigmine)

As rivastigmine in the present invention, rivastigmine (free form) showing an acetylcholineaterase inhibitory action and/or a pharmaceutically acceptable salt thereof can be used. Salts of rivastigmine include rivastigmine hydrochloride, rivastigmine tartrate and the like, and free rivastigmine is preferred in view of good transdermal permeability.

The content of rivastigmine in an adhesive agent layer can be appropriately adjusted depending on a desired therapeutic effect, and there is no particular limitation for the content. In a case where the acrylic adhesive agent in an adhesive agent layer is an adhesive agent (I) described below, rivastigmine may be contained in the adhesive agent layer in an amount of 0.1 mass % or more, preferably 1 mass% or more, more preferably 10 mass% or more. Further, in a case where the acrylic adhesive agent in an adhesive agent layer is the adhesive agent (II) described below, rivastigmine may be contained in the adhesive agent layer in an amount of 0.1 mass % or more, preferably 1 mass% or more, more preferably 10 mass% or more. Rivastigmine has good compatibility with an adhesive agent in the adhesive agent layer in the present invention. Therefore, even in a case where the content of rivastigmine is high, cold flow (a phenomenon in which an adhesive agent layer becomes soft and undergoes flow deformation in an environment such as ordinary temperature) and bleeding (a phenomenon in which a component flows out of the adhesive agent layer) can be suppressed. Further, the acrylic adhesive agent described below is contained in the adhesive skin patch according to the present invention. Therefore, even in a case the content of rivastigmine in the adhesive skin patch is relatively high, and the content of the adhesive agent is relatively low, reduction in adhesiveness of the adhesive skin patch can be suppressed.

In a case where the acrylic adhesive agent in an adhesive agent layer is the adhesive agent (I) described below, rivastigmine may be contained in the adhesive agent layer in an amount of 40 mass% or less, preferably less than 30 mass%, more preferably 25 mass% or less, most preferably 20 mass% or less. Further, in a case where the acrylic adhesive agent in an adhesive agent layer is the adhesive agent (II) described below, rivastigmine may be contained in the adhesive agent layer in an amount of 40 mass% or less, preferably less than 30 mass%, more preferably 25 mass% or less. Even in a case where the content of rivastigmine in the adhesive agent layer in the present invention is low, good transdermal permeability of rivastigmine can be achieved.

### (Adhesive agent)

The adhesive agent in the present invention comprises the adhesive agent (I) or the adhesive agent (II) described below. Since these adhesive agents have high adhesiveness to the skin and low skin irritation, and also have low reactivity with rivastigmine, the decrease in the transdermal absorption of rivastigmine can be suppressed.

### [Adhesive agent (I)]

The adhesive agent (I) is a resin mixture comprising an acrylic copolymer comprising at least diacetone acrylamide as a constituting component, preferably 100 parts by mass of the acrylic copolymer (A) described below and 0.1 to 30 parts by mass of the acrylic copolymer (B) described below or 0.05 to 2 parts by mass of a polyamine compound.

The acrylic copolymer (A): an acrylic copolymer comprising alkyl (meth)acrylate as a main monomer component and 3 to 45 mass% of diacetone acrylamide as an essential monomer component, but not comprising a free carboxyl group (a carboxy group).

The acrylic copolymer (B): an acrylic copolymer comprising alkyl (meth)acrylate as a main monomer component and a primary amino group and/or a carboxy hydrazide group in a side chain, but not comprising a free carboxyl group.

In the adhesive agent (I), fine network structures based on crosslinking reactions with a carbonyl group from diacetone acrylamide contained in the acrylic copolymer (A) and a primary amino group and a carboxy hydrazide groups contained in the acrylic copolymer (B) can be formed through the entire adhesive agent layer to hold rivastigmine and the like in the network structures.

The adhesive agent (I) can be prepared, for example, according to WO2011/027786 and Japanese Patent Application Laid-Open No. 2005-325101.

Specific methods of preparing the adhesive agent (I) include the following. In order to manufacture the acrylic copolymer (A), a method is illustrated comprising preparing alkyl (meth)acrylate as a main monomer component, and adding diacetone acrylamide which is also a monomer component to give 3 to 45 mass% relative to the total monomer, and performing radical polymerization. These monomer components can be polymerized by the conventional method using a polymerization initiator such as a peroxide compound or an azo based compound. When polymerizing these monomers, it is preferred to appropriately add a solvent in order to adjust the viscosity of a reaction solution.

As an alkyl (meth)acrylate, preferably used are alkyl (meth)acrylates having an alkyl group with 1 to 12 carbon atoms. Specifically, they include methyl (meth)acrylate, ethyl (meth)acrylate, propyl (meth)acrylate, butyl (meth)acrylate, 2-ethylhexyl (meth)acrylate, octyl (meth)acrylate, dodecyl (meth)acrylate and the like. These alkyl (meth)acrylates can be used alone or in combinations of 2 or more.

The acrylic copolymer (A) preferably has a number average molecular weight of 100000 to 1500000, and preferably has a weight average molecular weight of 300000 to 2500000. A molecular weight of the acrylic copolymer (A) within the above range is preferred since an adhesive agent layer of a rivastigmine-containing adhesive skin patch shows moderate adhesiveness to the skin. Within the above range, the number average molecular weight of the acrylic copolymer (A) is more preferably 300000 to 1000000, most preferably 500000 to 800000. Further, within the above range, the weight average molecular weight of the acrylic copolymer (A) is more preferably 500000 to 2000000, most preferably 1000000 to 1500000.

In order to manufacture the acrylic copolymer (B), a method is illustrated comprising preparing alkyl (meth)acrylate as a main monomer component, and adding a monomer component for introducing a primary amino group and/or a monomer component for introducing a carboxy hydrazide group to give 1 to 30 mass% relative to the total monomer, and performing radical polymerization, and then converting a side chain from the monomer component for introducing a carboxy hydrazide group into a carboxy hydrazide group. When performing radical polymerization of the monomer components, they may be polymerized by the conventional method using a polymerization initiator such as a peroxide compound or an azo based compound. When performing radical polymerization of the monomer components, it is preferred to appropriately add a solvent in order to adjust the viscosity of a reaction solution. Note that alkyl (meth)acrylate similar to those illustrated for the acrylic copolymer (A) can be used for manufacture of the acrylic copolymer (B).

Further, there are two or more, preferably three or more primary amino groups and/or carboxy hydrazide groups in one molecule chain of the acrylic copolymer (B) in order to show moderate crosslinking with the acrylic copolymer (A).

Further, a monomer component for introducing a primary amino group and/or a monomer component for introducing a carboxy hydrazide group and an alkyl (meth)acrylate monomer are preferably mixed in 1:5 to 1:100 by molar ratio to allow copolymerization.

Monomer components for introducing a primary amino group into the acrylic copolymer (B) include a compound having a vinyl group polymerizable with alkyl (meth)acrylate and having a primary amino group. Examples of such a compound include vinylamine and the like.

Monomer components for introducing a carboxy hydrazide group into the acrylic copolymer (B) include a compound having a vinyl group polymerizable with alkyl (meth)acrylate and having a keto group capable of reacting with a hydrazide compound. Examples of such a compound include diacetone acrylamide, acrolein, acetoacetoxylethyl methacrylate and the like.

In order to convert a side chain from a monomer component into a carboxy hydrazide group for introducing a carboxy hydrazide group, a polymer obtained from the above radical polymerization may be dissolved in a polar solvent to allow a dihydrazide of dicarboxylic acid to react in the presence of an acid catalyst. Examples of a dihydrazide of dicarboxylic acid include adipic acid dihydrazide, glutaric acid dihydrazide, pimelic acid dihydrazide and the like.

The acrylic copolymer (B) preferably has a number average molecular weight of 1500 to 50000, and preferably has a weight average molecular weight of 2000 to 100000. A molecular weight of the acrylic copolymer (B) at or above the lower limit described above is preferred since the gelation of a mixed liquid can be suppressed, leading to good coatability when producing an adhesive agent layer. Further, a molecular weight of the acrylic copolymer (B) at or below the upper limit described above is preferred since a moderate crosslinking state with the acrylic copolymer (A) can be obtained. Within the above range, the number average molecular weight of the acrylic copolymer (B) is more preferably 2000 to 10000, most preferably 3000 to 8000. Further, within the above range, the weight average molecular weight of the acrylic copolymer (B) is more preferably 5000 to 20000, most preferably 8000 to 15000.

For the adhesive agent (I), a resin mixture comprising 100 parts by mass of the acrylic copolymer (A) and 0.05 to 2 parts by mass of a polyamine compound can be used as an adhesive agent. Polyamine compounds include polyhydrazide compounds (adipic acid dihydrazide and the like) described in WO2011/027786. These compounds can be prepared according to the above disclosure.

There is no particular limitation for the content of the adhesive agent (I) in an adhesive agent layer, but it may be 50 to 95 mass% in the adhesive agent layer, preferably 60 to 95 mass%, more preferably 60 to 90 mass%.

### [Adhesive agent (II)]

The adhesive agent (II) is an acrylic copolymer comprising at least acetoacetoxyalkyl (meth)acrylate as a constituting component, preferably is a copolymer of acetoacetoxyalkyl (meth)acrylate and one or more vinyl monomers polymerizable with the above acetoacetoxyalkyl (meth)acrylate, the copolymer not comprising a free carboxyl group.

The adhesive agent (II) can form fine network structures based on crosslinking reactions between acetoacetoxyalkyl groups contained in the copolymer through the entire adhesive agent to hold rivastigmine and the like in the above network structures.

The adhesive agent (II) can be prepared, for example, according to Japanese Patent No. 3809462. For example, a copolymer of acetoacetoxyalkyl (meth)acrylate and other vinyl monomers can be manufactured by the conventional method using a polymerization initiator such as a peroxide compound or an azo based compound.

Examples of acetoacetoxyalkyl (meth)acrylate include those in which one hydroxyl group in alkylene glycols is acylated with an acetoacetyl group, and the other hydroxy group is acylated with acrylic acid or methacrylic acid. As such a compound, for example, 2-acetoacetoxylethyl methacrylate, 2-acetoacetoxyethyl acrylate and the like are preferably used, and 2-acetoacetoxylethyl methacrylate is more preferably used. Further, the content of acetoacetoxyalkyl (meth)acrylate is preferably about 5 to 50 mass%, more preferably about 10 to 45 mass% when the total mass of copolymers is taken as 100.

Examples of a vinyl monomer copolymerizable with acetoacetoxyalkyl (meth)acrylate include a compound having a vinyl group in the molecule thereof. Such compounds include alkyl (meth)acrylates having an alkyl group with 1 to 12 carbon atoms; functional monomers having a functional group such as a hydroxyl group, an amide group and an alkoxylalkyl group in the molecules thereof; and polyalkyleneglycol di(meth)acrylates and the like. These compounds can be used alone or in combinations of two or more.

Specific examples of alkyl (meth)acrylate having an alkyl group with 1 to 12 carbon atoms include methyl (meth)acrylate, ethyl (meth)acrylate, propyl (meth)acrylate, butyl (meth)acrylate, 2-ethylhexyl (meth)acrylate, octyl (meth)acrylate, dodecyl (meth)acrylate and the like. Further, specifically, functional monomers having functional groups in the molecules thereof include 2-methoxyethyl (meth)acrylate, diacetone acrylamide, 2-hydroxyethyl (meth)acrylate and the like. Moreover, specifically, polyalkyleneglycol di(meth)acrylates include diethyleneglycol di(meth)acrylate, triethyleneglycol di(meth)acrylate, triethyleneglycol di(meth)acrylate, tetrethyleneglycol di(meth)acrylate and the like.

There is no particular limitation for the content of the adhesive agent (II) in an adhesive agent layer, but it may be 50 to 95 mass% in the adhesive agent layer, preferably 60 to 90 mass%, even more 60 to 85 mass%.

In a case where a mixture of the adhesive agent (I) and the adhesive agent (II) is used as an acrylic adhesive agent in an adhesive agent layer, the total amount of the adhesive agent in the adhesive agent layer may be 50 to 95 mass%, preferably 60 to 95 mass%, more preferably 60 to 90 mass%.

### (Other components)

Other components may be contained in the adhesive agent layer used for the present invention if desired. These components include an antioxidizing agent (anti-oxidant), a tackifier, a plasticizing agent, an antiseptic agent, a pH adjuster, a chelating agent, a transdermal absorption promoter, an excipient, a flavoring agent, a coloring agent, fatty acid, fats and oils, cholesterol and the like.

Antioxidizing agents (anti-oxidants) include tocopherol (α-tocopherol and the like) and esters thereof, ascorbic acid, ascorbyl palmitate, dibutylhydroxytoluene, butylated hydroxyanisole, propyl gallate, 1,3-butylene glycol, benzoic acid and the like.

Note that methods for preventing oxidation of a drug include, in addition to a method comprising blending the aforementioned antioxidizing agent in an adhesive agent layer, a method comprising enclosing a deoxygenating agent in a packaging bag, a method comprising including a deoxygenating agent in a release liner, a support or a packaging bag, a method comprising decompressing the inside of a packaging bag to reduce air, a method comprising purging the inside of a packaging bag with an inert gas such as nitrogen gas and then sealing an adhesive skin patch and a method comprising layering a release liner (such as an oxygen impermeable liner) which can increase drug stability on an adhesive skin patch and the like. These methods can be appropriately used if desired.

There is no particular limitation for the content of other components in an adhesive agent layer, but the total amount of other components in an adhesive agent layer may be 0.01 to 30 mass%, preferably 0.05 to 20 mass%, more preferably 0.1 to 15 mass%.

The adhesive agent layer used for the present invention may comprise rivastigmine and an acrylic adhesive agent, but not a plasticizing agent and the like. According to the present invention, even in a case where a plasticizing agent and the like are not contained in an adhesive agent layer, the solubility of a drug and/or the compatibility between an adhesive agent and a drug are excellent. Therefore, an adhesive agent layer can be well formed. Further, skin irritation is low, and transdermal permeability of rivastigmine is excellent. Further, according to the present invention, even in a case where a plasticizing agent and the like are not contained in an adhesive agent layer, the solubility of a drug and/or the compatibility between an adhesive agent and a drug are excellent. Therefore, an adhesive agent layer can be obtained in which bleeding is suppressed and skin adhesiveness is also good.

### [Support]

The support used for the present invention is preferred to be impermeable or poorly-impermeable into a drug component, and is also preferred to be soft. Specifically, supports include a resin film of polyethylene, polypropylene, ethylenevinylacetate copolymer, ethylene-vinyl acetate-carbon monoxide copolymer, ethylene-butyl acrylate-carbon monoxide copolymer, nylon, polyester, polyethylene terephthalate (PET), polybutylene terephthalate and the like; and an aluminum sheet: and the like. These may be layered to form a sheet, or may be layered together with a woven fabric or a non-woven fabric. As a support, those comprising polyethylene terephthalate (PET) is preferred in that the stability of rivastigmine can be increased (specifically, in that the production of rivastigmine analogs can be suppressed). For example, preferred supports comprising polyethylene terephthalate can include a layered product of a PET non-woven fabric and a PET film and the like.

In order to increase adhesiveness with an adhesive agent layer, surface treatment such as corona treatment and plasma discharge treatment may be performed on the surface of a support, or anchor coating treatment may also be performed with an anchoring agent.

A release sheet for protecting a surface of an adhesive agent layer may be provided on the surface of the adhesive agent layer opposite to a support. When a user of an adhesive skin patch removes a release sheet to expose the surface of an adhesive agent layer, the adhesive skin patch becomes ready to use. Preferably, the raw material of a release sheet can be easily removed from an adhesive agent layer, and does not allow permeation of an active ingredient. Such raw materials include, for example, a single layer film selected from raw materials such as a plastic film of polyethylene, polyester, polyethylene terephthalate (PET), polyvinyl chloride, or polypropylene and a metal film and the like; a layered film in which two or more raw materials are layered. Those in which the surfaces of these films are siliconized can also be used.

### [Method of preparing adhesive skin patch]

There is no particular limitation for the method of preparing the adhesive skin patch according to the present invention, and it can be prepared by using a preparation method of a known adhesive skin patch. For example, the adhesive skin patch according to the present invention can be obtained by uniformly stirring components constituting an adhesive agent layer, and dry-coating the resulting composition on a surface of a support using a coater. Note that in order to increase the anchoring properties between an adhesive agent layer and a support, a known anchoring layer may be provided on a surface of the adhesive agent layer. In that case, the adhesive agent layer and the anchoring layer may together form an adhesive agent layer. Further, the adhesive agent layer may comprise a single adhesive agent layer, or may comprise two or more adhesive agent layers.

The adhesive skin patch according to the present invention may be prepared as an adhesive skin patch having a layered structure with three or more layers comprising a support, a drug layer, an adhesive agent layer and the like by providing the adhesive agent layer and the drug layer separately as in the conventional adhesive skin patch. Alternatively, it may be prepared as an adhesive skin patch comprising a support and an adhesive agent layer only (i.e., an adhesive skin patch not having a layered structure of two layers or more) since the adhesive agent layer used for the present invention has good compatibility with a drug and the like and excellent adhesiveness. Note that an adhesive skin patch comprising the aforementioned release sheet on a surface of an adhesive agent layer opposite to a support in addition to the support and a drug layer is also included in an "adhesive skin patch comprising a support and an adhesive agent layer only". In a case where the adhesive skin patch according to the present invention is prepared as an adhesive skin patch comprising a support and an adhesive agent layer only, preparation can be performed via a simple manufacturing process as compared with the conventional adhesive skin patch having a layered structure with three or more layers in which an adhesive agent layer and a drug layer are provided separately.

### [Adhesive skin patch according to the present invention]

The adhesive skin patch according to the present invention has excellent transdermal permeability of rivastigmine. The transdermal permeability of rivastigmine can be evaluated by conducting a known transdermal permeability test to measure the transdermal permeation amount and/or transdermal permeation rate of rivastigmine.

As described above, since the adhesive skin patch according to the present invention has excellent transdermal permeability of rivastigmine and the like, rivastigmine can be transdermally absorbed into the body in an appropriate fashion. Therefore, the adhesive skin patch according to the present invention can be suitably used to treat or prevent Alzheimer type dementia since rivastigmine can be simply administered.

Moreover, the adhesive skin patch according to the present invention has excellent adhesiveness. The adhesiveness of an adhesive skin patch can be evaluated by measuring the peel force of the adhesive skin patch.

### EXAMPLES

The present invention will be described in detail with reference to the following Examples. However, these Examples are merely intended to illustrate the present invention, and shall not limit the scope of the present invention.

The acrylic adhesive agents used in Examples herein are the adhesive agent (I) or the adhesive agent (II) as described below.

### Adhesive agent (I)

A solution of the copolymer (A) and a solution of the copolymer (B) obtained by the synthesis method described below were mixed in a mass ratio of 100:5 (the copolymer (A):the copolymer (B)) to obtain an adhesive agent (I).

### [Copolymer (A) for adhesive agent (I)]

Added and mixed were 200 parts by mass of 2-ethylhexyl acrylate, 100 parts by mass of butyl acrylate, 50 parts by mass of diacetone acrylamide and 300 parts by mass of ethyl acetate. This mixture was fed to a separable flask equipped with a stirrer and a reflux condenser, and heated to 75°C while stirring under nitrogen purge. A solution in which 2 parts by mass of benzoyl peroxide was dissolved in 20 parts by mass of ethyl acetate was aliquoted into five, and one aliquot was added to the separable flask to initiate polymerization. The remaining 4 aliquots were added one aliquot every one hour interval starting 2 hours after the initiation of the reaction, and allowed to react for another 2 hours after the end of addition. Note that 50 parts by mass of ethyl acetate was added 4 times every 2 hours after the initiation of the reaction to adjust viscosity. After the end of the reaction, it was cooled, and then ethyl acetate was added to obtain a copolymer (A) used for the adhesive agent (I) having a solid content concentration of 30 mass%.

### [Copolymer (B) for adhesive agent (I)]

Added and mixed were 660 parts by mass of ethyl acrylate, 70 parts by mass of diacetone acrylamide, 40 parts by mass of dodecylmercaptan as a molecular weight modifier and 400 parts by mass of ethyl acetate. This mixture was fed to a separable flask equipped with a stirrer and a reflux condenser, and heated to 70°C while stirring under nitrogen purge. A solution in which 5 parts by mass of azobisisobutyronitrile was dissolved in 100 parts by mass of ethyl acetate was aliquoted into five, and one aliquot was added to the separable flask to initiate polymerization. The remaining 4 aliquots were added one aliquot every one hour interval starting 2 hours after the initiation of the reaction, and allowed to react for another 2 hours after the end of addition. Note that 50 parts by mass of ethyl acetate was added 3 times every 2 hours after the initiation of the reaction to adjust viscosity. Then, a solution in which 40 parts by mass of adipic acid dihydrazide was dissolved in a mixed liquid of 40 parts by mass of purified water, 1600 parts by mass of methanol and 260 parts by mass of ethyl acetate was added, and 5 parts by mass of concentrated hydrochloric acid was further added, and then heated to 70°C. After the completion of the reaction, it was cooled and washed 3 times with purified water, and then the product was dissolved in a mixed solvent of 700 parts by mass of ethyl acetate, 1400 parts by mass of acetone and 400 parts by mass of methanol to obtain a copolymer (B) used for the adhesive agent (I) having a solid content concentration of 30 mass%.

### Adhesive agent (II)

Uniformly dissolved to prepare a monomer liquid were 158 parts by mass of 2-ethylhexyl acrylate, 35.1 parts by mass of 2-acetoacetoxylethyl methacrylate, 76.2 parts by mass of methyl methacrylate, 80.3 parts by mass of diacetone acrylamide and 1.0 part by mass of tetraethyleneglycol dimethacrylate. To a 2-liter 4-neck flask equipped with a Dimroth condenser, a thermometer, a nitrogen gas blowing pipe and an impeller, introduced were 100 parts by mass of this monomer liquid. Then 350 parts by mass of ethyl acetate was added as a solvent. Temperature was increased to 75°C while blowing nitrogen gas at a flow rate of 100 mL/min, and maintained at 75°C for 30 minutes. Then 0.35 parts by mass of benzoyl peroxide as an initiator was dissolved in 5 parts by mass of ethyl acetate, and the resulting solution was added. Outside temperature was then set to 85°C. After confirming reflux of solvent, the remaining monomer liquid was continuously introduced over 3 hours. Starting at 1 hour after the start of continuous introduction of the monomer liquid, 500 parts by mass of ethyl acetate was introduced over 3 hours. Stirring was continued for 12 hours after introducing ethyl acetate, and then 0.5 parts by mass of benzoyl peroxide was introduced as an additional catalyst, and heat-treated for 12 hours, and then cooled to obtain the adhesive agent (II).

### Example 1

### (Preparation of adhesive skin patch)

Compositions were produced according to the compositions shown in Table 1. The formulation according to the present invention was dry-coated (60°C, 15 minutes) on a support (a layered product film of a PET non-woven fabric and a PET film was used) to obtain an adhesive skin patch. Further, as the conventional formulation, a commercially available product (Novartis Pharma K. K.) in which a drug layer and an adhesive agent layer are provided on a support was used. In Table 1, Duro tack 387-2051 (Henkel Japan, Ltd.) and EUDRAGIT E100 (Alternative name: EUDRAGIT E100, Degussa Japan Co., Ltd. / Higuchi, Inc.) are acrylic adhesive agents. Note that "%" refers to mass% in Table 1.

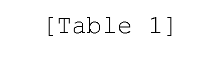

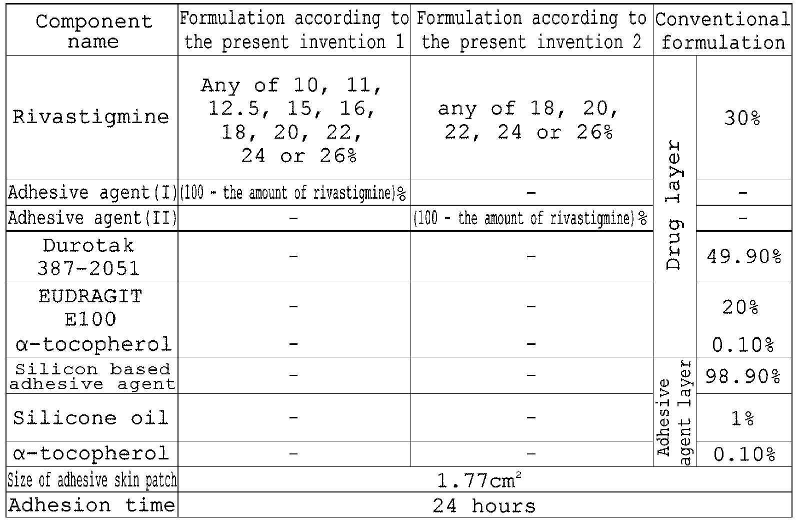

### (Studies on the transdermal permeation amount of rivastigmine)

The skin of a hair-less mouse was punched out into discs with a diameter of 24 mm, and the resulting skin pieces were placed in a 6-well cell culture plate (each well contained 1.2 mL of PBS (pH7.4) as a receiver solution) with the dermal layer facing the well side. An adhesive layer of an adhesive skin patch punched out into a disc with a diameter of 10 mm (the concentration of rivastigmine was any of 10, 11, 12.5, 15, 16, 18, 20, 22, 24 or 26 mass%) was patched on the side of the horny layer of the skin. A temperature condition was maintained at 32°C, and over time, receiver solutions were collected multiple times starting at the skin placement for over 24 hours. The concentration of rivastigmine in the collected receiver solution was measured by HPLC, and based on the numerical value obtained, the cumulative transdermal permeation amount of rivastigmine per skin area at 24 hours after adhesion was computed.

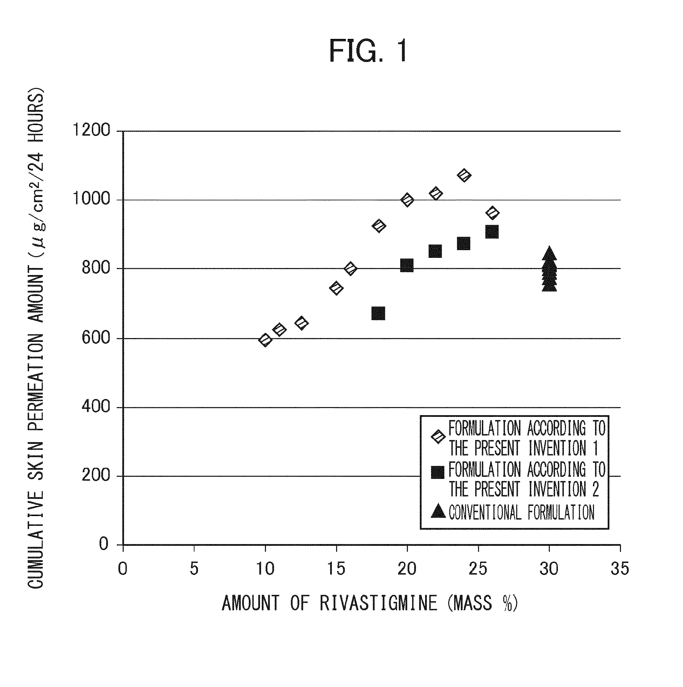

The evaluation results of the cumulative transdermal permeation amount of rivastigmine are shown in Fig. 1 (the mean value ± SD, n= 3). As shown in Fig. 1, the adhesive skin patch according to the present invention showed a similar cumulative transdermal permeation amount as the conventional adhesive skin patch, or a cumulative transdermal permeation amount higher than the conventional adhesive skin patch even in a case where the concentration of rivastigmine in an adhesive skin patch was low.

### Example 2

Compositions were produced according to the compositions shown in Table 2. The formulation according to the present invention was dry-coated (60°C, 15 minutes) on a support (a layered product film of a PET non-woven fabric and a PET film was used) to obtain an adhesive skin patch. Further, as the conventional formulation, a commercially available product (Novartis Pharma K. K.) in which a drug layer and an adhesive agent layer are provided on a support was used. In Table 2, Duro tack 387-2051 (Henkel Japan, Ltd.) and EUDRAGIT E100 (Alternative name: EUDRAGIT E100, Degussa Japan Co., Ltd. / Higuchi, Inc.) are acrylic adhesive agents. Note that "%" refers to mass% in Table 2. The resulting adhesive skin patch was patched on the skin, and the adhesiveness of the adhesive skin patch after 24 hours was measured according to the following test method. Results are shown in Table 3.

### (Test method of peel force of adhesive skin patch)

Each adhesive skin patch was cut into a 20-mm width, and then patched on the skin removed from a Yucatan micro pig, and press-fit with one stroke of a 2 kg roller. After allowing it to stand for 24 hours under conditions of 32°C (and a humidity of 60%), the adhesive skin patch was peeled off from the skin with a pull test machine at a rate of 300 mm/min, and the mean of the maximum load and the minimum load upon peeling was taken as the peel force.

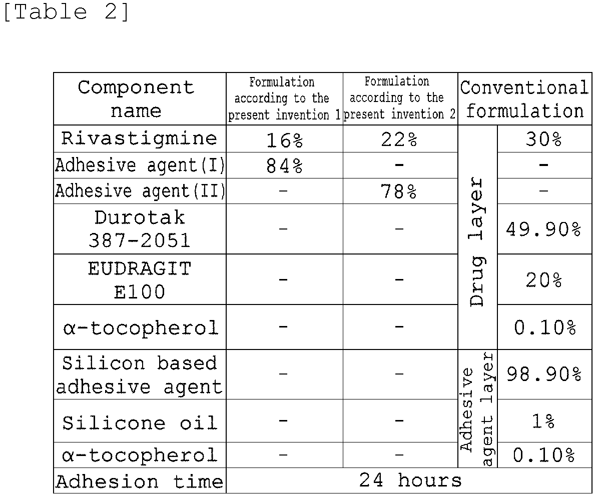

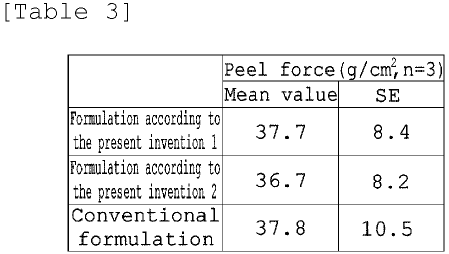

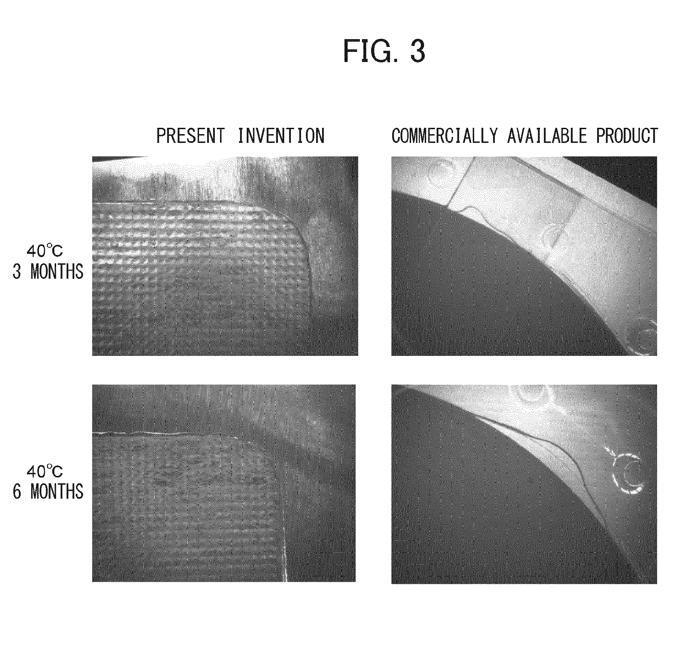

As shown in Fig. 3, it was observed that even at 24 hours after adhesion, the adhesive skin patch according to the present invention showed a peel force similar to that of the conventional adhesive skin patch, and had excellent adhesiveness similar to that of the conventional adhesive skin patch.

### Example 3

### (Preparation of adhesive skin patch)

Compositions were produced according to the compositions shown in Table 4. The formulation according to the present invention was dry-coated (60°C, 15 minutes) on a support (a layered product film of a PET non-woven fabric and a PET film was used) to obtain an adhesive skin patch. Further, as the conventional formulation, a commercially available product (Novartis Pharma K. K.) in which a drug layer and an adhesive agent layer are provided on a support was used. In Table 4, Duro tack 387-2051 (Henkel Japan, Ltd.) and EUDRAGIT E100 (Alternative name: EUDRAGIT E100, Degussa Japan Co., Ltd. / Higuchi, Inc.) are acrylic adhesive agents. Note that "%" refers to mass% in Table 4.

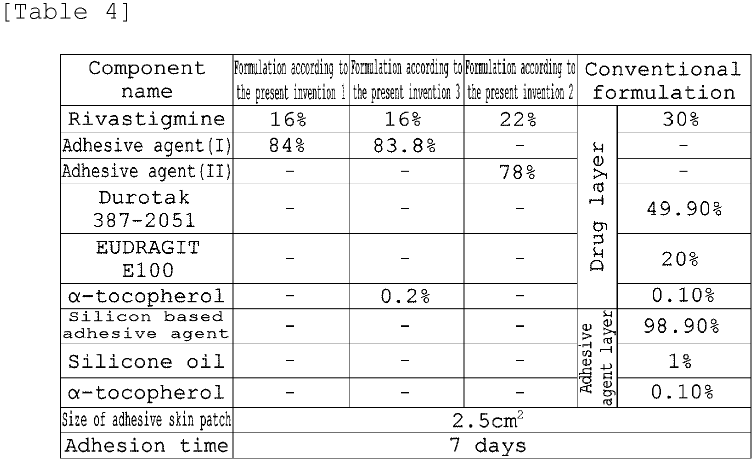

### (Studies on skin irritation)

The skin irritation of an adhesive skin patch was studied according to the following procedure. Using non-woven fabric adhesive dressings, the adhesive skin patch was fixed on the back of a rabbit whose back hair was cut. The adhesive skin patch was removed 6 hours after adhesion, and an application site on which the adhesive skin patch was adhered (hereinafter referred to as an "application site") was lightly wiped off with absorbent cotton wetted with lukewarm water, and allowed to stand for 30 minutes, and then the application site was observed. After the end of observation, a new adhesive skin patch was fixed to the same site as the initial application site, and the same operation was repeated for seven days. Further, 48 hours and 72 hours after removing the adhesive skin patch on Day 7 (that is, the last adhesive skin patch), the application site was also observed in a similar fashion. Observation was performed based on the evaluation criteria A and B according to Draize et al. as described below.

### (Evaluation criteria according to Draize et al.)

Criterion A: formation of erythema and scab
No erythema = Score 0
Very mild erythema = Score 1
Obvious erythema = Score 2
Moderate to serious erythema = Score 3
Serious erythema to mild scab formation = Score 4
Criterion B: Formation of edema
No edema = Score 0
Very mild edema = Score 1
Mild edema = Score 2
Moderate edema (about 1 mm of swelling) = Score 3
Serious edema = Score 4

For each adhesive skin patch, scores based on the above criteria A and B were recorded, and the mean score was computed for each adhesive skin patch based on the following expression. The mean score = [(the total score of Criterion A) + (the total score of Criterion B)] / 5

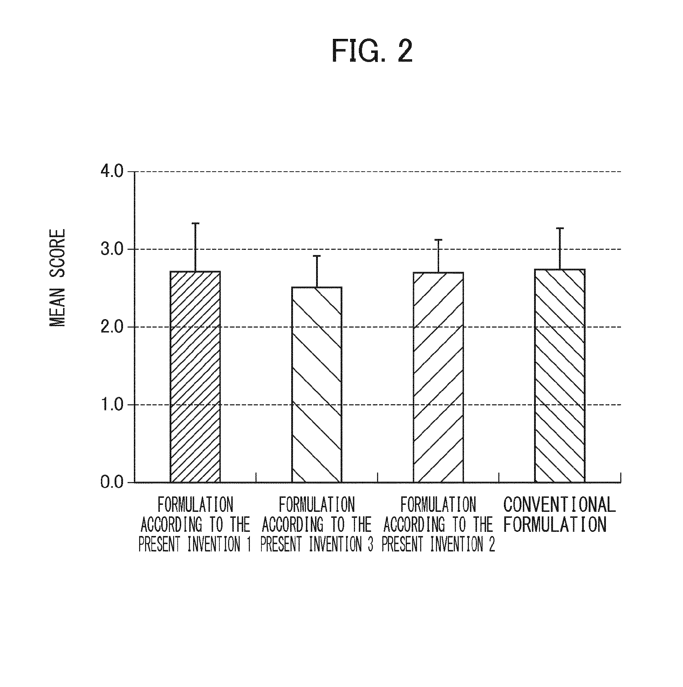

As shown in Fig. 2, the skin irritation due to the adhesive skin patch according to the present invention was suppressed to a similar extent as the conventional adhesive skin patch. Further, the adhesive skin patches according to the present invention studied in Examples herein showed a good followability even though a plasticizing agent was not contained in the adhesive agent layers. Further, for the adhesive skin patches from Examples herein, bleeding was not observed at all.

### (Cold flow resistance test)

An adhesive skin patch was prepared based on the "formula 1 according to the present invention" in Example 3, and the adhesive skin patch in which a release sheet (a PET film) was provided on the side of the adhesive agent layer was placed into a packaging bag, and stored at 40°C. Further, a commercially available product of a rivastigmine-containing adhesive skin patch (Novartis Pharma K.K.) was stored at 40°C. At both 3 months and 6 months after the start of storage, each adhesive skin patch was removed from the packaging bag, and the condition of the adhesive agent layer was visually inspected.

As shown in the left column of Fig. 3, in the adhesive skin patch according to the present invention, the adhesive agent layer of the adhesive skin patch did not show flow deformation both at 3 months and 6 months after the start of storage, and cold flow was not observed. In contrast, as shown in the right column of Fig. 3, in the commercially available product, the adhesive agent layer flowed out to the outside of the adhesive skin patch both at 3 months and 6 months after the start of storage, and the adhesive skin patch strongly adhered to the packaging bag, and cold flow was observed.

## Claims

1. An adhesive skin patch comprising a support and an adhesive agent layer arranged on the support, wherein
the adhesive agent layer comprises at least
rivastigmine and/or a pharmacologically acceptable salt thereof, and
an acrylic adhesive,
wherein the acrylic adhesive agent comprises an adhesive agent (I) or an adhesive agent (II),
the adhesive agent (I) being an acrylic copolymer comprising at least diacetone acrylamide as a constituting component,
the adhesive agent (II) being an acrylic copolymer comprising at least acetoacetoxyalkyl (meth)acrylate as a component.

2. The adhesive skin patch according to claim 1, wherein the acrylic adhesive agent comprises an adhesive agent (I) or an adhesive agent (II),
the adhesive agent (I) being a resin mixture comprising 100 parts by mass of an acrylic copolymer (A) and 0.1 to 30 parts by mass of an acrylic copolymer (B) or 0.05 to 2 parts by mass of a polyamine compound,
the acrylic copolymer (A) being an acrylic copolymer comprising alkyl (meth)acrylate as a main monomer component and 3 to 45 mass% of diacetone acrylamide as an essential monomer component, but not comprising a free carboxyl group,
the acrylic copolymer (B) being an acrylic copolymer comprising alkyl (meth)acrylate as a main monomer component and a primary amino group and/or a carboxy hydrazide group in a side chain, but not comprising a free carboxyl group,
the adhesive agent (II) being a copolymer of acetoacetoxyalkyl (meth)acrylate and one or more vinyl monomers copolymerizable with the acetoacetoxyalkyl (meth)acrylate, the copolymer not comprising a free carboxyl group.

3. The adhesive skin patch according to claim 1 or 2, comprising the support and the adhesive agent layer only.

4. The adhesive skin patch according to claim 1 or 2, wherein the adhesive agent layer is a single layer.

5. The adhesive skin patch according to any one of claims 1 to 4 for use in treatment or prevention of Alzheimer type dementia.
